Europäisches Patentamt

European Patent Office

Office européen des brevets

(19)

(11) Veröffentlichungsnummer: **0 068 218**
**B1**

(12) # EUROPÄISCHE PATENTSCHRIFT

(45) Veröffentlichungstag der Patentschrift:
10.10.84

(21) Anmeldenummer: 82105099.4

(22) Anmeldetag: 11.06.82

(51) Int. Cl.³: **C 07 C 43/04**, C 07 C 41/06,
C 07 C 7/148 // C07C41/36,
C07C29/76

(54) **Verfahren zur Herstellung von reinen Alkyl-tert.-alkylethern und weitgehend vom Isoolefin und vom Alkanol befreiten Kohlenwasserstoff-Raffinaten.**

(30) Priorität: 19.06.81 DE 3124293

(43) Veröffentlichungstag der Anmeldung:
05.01.83 Patentblatt 83/1

(45) Bekanntmachung des Hinweises auf die Patenterteilung:
10.10.84 Patentblatt 84/41

(84) Benannte Vertragsstaaten:
AT BE DE FR GB IT NL

(56) Entgegenhaltungen:
DE - A - 2 629 769
DE - A - 2 802 198
DE - A - 2 938 222
FR - A - 2 448 521

(73) Patentinhaber: BAYER AG, Konzernverwaltung RP
Patentabteilung, D-5090 Leverkusen 1 Bayerwerk (DE)
Patentinhaber: EC ERDÖLCHEMIE GMBH,
Postfach 75 20 02, D-5000 Köln 71 (DE)

(72) Erfinder: Herwig, Jens, Dr., Auf dem Hügel 23,
D-5000 Koeln 41 (DE)
Erfinder: Scheef, Hans-Volker, Goethestrasse 69,
D-4047 Dormagen 1 (DE)
Erfinder: Schleppinghoff, Bernhard, Dr.,
Adolf-Kolping-Strasse 5, D-4047 Dormagen 1 (DE)
Erfinder: Lange, Peter Michael, Dr.,
Walter-Flex-Strasse 9, D-5090 Leverkusen 1 (DE)

## Beschreibung

Die vorliegende Erfindung betrifft ein Verfahren zur Herstellung von reinen Alkyl-tert.-alkylethern und weitgehend vom Isoolefin und vom Alkanol befreiten Kohlenwasserstoff-Raffinaten durch Umsetzung von Alkanolen und mindestens 1 tert.-Olefin enthaltenden Kohlenwasserstoff-Gemischen an sauren Ionenaustauschern und absorptiver Entfernung von überschüssigem Alkanol aus dem Reaktionsgemisch.

Alkyl-tert.-alkylether, wie Methyl-tert.-butylether (MTBE) und tert.-Amyl-methylether (TAME), dienen beispielsweise als Vergaserkraftstoff-Additive zur Oktanzahlverbesserung und als Löse- und Extraktionsmittel, die beispielsweise im Gegensatz zu vielen anderen Ethern fast keine Peroxide bilden. Außerdem eignen sich die Alkyl-tert.-Alkylether als Ausgangsstoffe zur Darstellung der reinen, ihnen zugrundeliegenden tert.-Olefine über die Etherspaltung.

Es ist bekannt, tert.-Olefine und Alkanole an sauren Ionenaustauschern zu Alkyl-tert.-alkylethern umzusetzen (DE-PS 1 224 294). Da diese Reaktion sehr selektiv ist, eignen sich als Einsatzstoffe besonders auch Gemische von tertiären Olefinen mit anderen Kohlenwasserstoffen, wie sie beispielsweise in thermischen oder katalytischen Crackern anfallen.

Je nach Art des tertiären Olefins und nach Wahl des Alkanols läßt sich der Umsatz des tertiären Olefins verschieden gut an den quantitativen Umsatz annähern. Besonders hoch liegen dabei die Umsätze von i-Buten, beispielsweise aus dem $C_4$-Raffinat der thermischen Crackung (meist als $C_4$-Raffinat I bezeichnet), während die Umsätze von i-Amylen und anderen tertiären Olefinen mit steigender Kohlenstoffzahl unter vergleichbaren Bedingungen niedriger liegen. Durch spezielle Maßnahmen in der Reaktionsführung bei der Herstellung von MTBE, wie die Wahl von 2 oder mehreren hintereinandergeschalteten Reaktionsstufen (DE-OS 2 521 964, DE-OS 2 706 465, DE-OS 2 944 914) oder den Einsatz großer Methanolüberschüsse (DE-OS 2 853 769) lassen sich Umsatz und Ausbeute verbessern.

Besonders bei $C_4$-Raffinaten wird ein hoher i-Buten-Umsatz angestrebt, da zur Weiterverwertung des i-Buten-freien $C_4$-Raffinats der Restgehalt an i-Buten unter 2 Gew.-%, nach Möglichkeit sogar unter 1 oder unter 0,5 Gew.-% liegen soll. Diese Spezifikation ist erforderlich, wenn beispielsweise Maleinsäureanhydrid, Methylethylketon, Buten-1 oder Oktene durch Dimerisierung aus diesen meist als $C_4$-Raffinat II bezeichneten Restgemischen hergestellt werden sollen. Unter diesem Gesichtspunkt der Weiterverwendung muß außerdem dafür gesorgt werden, daß im $C_4$-Raffinat II kein Alkanol mehr vorliegt. Insofern werden an das Erreichen eines spezifikationsgerechten Raffinats und eines reinen Ethers, wie beispielsweise bei den Produktionsprozessen von MTBE und TAME, besondere Anforderungen gestellt.

Aufgrund azeotroper Effekte hat sich jedoch gezeigt, daß durch einfache Destillation die beiden geforderten Ziele, nämlich ein methanolfreier Ether und gleichzeitig ein methanolfreies $C_4$-Raffinat II oder $C_5$-Raffinat nicht erreicht werden können (vgl. DE-OS 2 802 198, Seite 3, Absatz 3). Besonders $C_4$- und $C_5$-Kohlenwasserstoffe bilden mit Methanol Azeotrope, die im Falle einer gemeinsamen i-Buten/i-Amylen-Veretherung, beispielsweise aus Kohlenwasserstoffen, die verschiedene $C_4$- und $C_5$-Olefine und -Alkane enthalten, eine exakte $C_4-C_5$-Trennung unmöglich machen, da mit dem Methanol auch $C_5$-Kohlenwasserstoffe in das $C_4$-Kopfprodukt des Debutanizers gelangen.

Ebenso bilden beispielsweise MTBE und Methanol ein Azeotrop. Zur Entfernung des Methanols aus dem $C_4$-Raffinat II und aus dem MTBE/Methanol-Azeotrop wurde als brauchbare Methode eine Wasserwäsche vorgeschlagen (DE-OS 2 246 004, US-PS 3 726 942). Für die Trennung des MTBE/Methanol-Azeotrops eignen sich außerdem eine Extraktivdestillation mit Dimethylsulfoxid (JP-OS 73-00 509) oder mit Pentan (US-PS 3 940 450) oder die destillative Abtrennung des Azeotrops vom reinen MTBE durch Verwendung höherer Drücke von 5 bis 20 bar (DE-OS 2 629 769).

Die Methanolabtrennung aus dem $C_4$-Raffinat II wird in der FR-PS 2 448 521 als Absorptionsprozeß am Molekularsieb mit anschließender Desorption durch heißen Stickstoff und wiedergewinnende Methanolkondensation aus dem $N_2$-Strom beschrieben.

Die genannten Maßnahmen zur Reingewinnung der Reaktionsprodukte und Raffinate verlangen wesentlich höhere Investitionen als der eigentliche Reaktionsteil der Veretherung und bedingen weiterhin, bezogen auf den Gesamtprozeß, einen großen zusätzlichen Energieverbrauch.

Aus US-PS 3 409 691 ist weiterhin bekannt, an makroporösen Kationenaustauschern in der $Na^+$-Form eine Menge von 500 ppm sekundäres Butanol von n-Hexan abzutrennen. Zur Regenerierung des Kationenaustauschers wird das Butanol durch Methanol verdrängt und das Methanol seinerseits durch Erhitzen des Kationenaustauschers auf 110° C über Nacht im Vakuum wieder entfernt.

Die DE-OS 2 027 066 beschreibt weiterhin die Möglichkeit mit Hilfe eines Nitrogruppen-haltigen Styrol-Divinylbenzol-Polymerisats Methanol aus Wasser (1 g Methanol pro 1 l Wasser) zu entfernen. Die Adsorptionsfähigkeit beträgt hierbei 18 g Methanol pro kg Adsorbens. Das Adsorbens wird mittels Dampfdestillation regeneriert.

Es wurde nun ein Verfahren zur Herstellung von reinen Alkyl-tert.-alkylethern und weitgehend vom tert.-Olefin und vom Alkanol befreiten Kohlenwasserstoff Raffinaten durch Umsetzung eines Alkanols und mindestens 1 tert.-Olefin enthaltenden Kohlenwasserstoffgemischen an sauren Kationenaustauschern gefunden, das dadurch gekennzeichnet ist, daß man überschüssiges, nicht umgesetztes Alkanol aus dem aus der Reaktionszone ablaufenden Produktgemisch oder aus seinen in der Aufarbeitung anfallenden Teilströmen durch Absorption an einem Absorberharz entfernt und das in die Reaktionszone einzuführende Alkanol mindestens teilweise dem hiermit beladenen Absorberharz durch Desorption mit Hilfe des mindestens 1 tert.-Olefin enthaltenden Kohlenwasserstoffgemisches entnimmt, wobei die in der Absorption und Desorption behandelten Stoffgemische in flüssiger Phase vorliegen.

Das zur Absorption und Desorption eingesetzte Absorberharz wird zur Durchführung des erfindungsgemäßen Verfahrens in mindestens 2 Schichten aufgeteilt, von denen mindestens eine im weitgehend alkanolfreien Zustand zur Absorption eingesetzt wird, während eine andere bereits mit dem Alkanol beladene Absorberschicht in den Strom der Einsatz-Kohlenwasserstoffe für das erfindungsgemäße Verfahren zur Desorption geschaltet wird. Rechtzeitig vor Erreichen einer solchen Beladung mit Alkanol, bei der das »Durchschlagen« des Alkanols durch die Absorberschicht noch mit Sicherheit vermieden werden kann, werden die genannten beiden Absorberschichten umgeschaltet, so daß die nunmehr beladene Schicht in den Strom der Einsatz-Kohlenwasserstoffe geschaltet wird und die vorher vom Alkanol befreite Absorberschicht für die Trennung des Alkanols aus den Endprodukten zur Verfügung steht. Selbstverständlich kann man auch mehr als 2 Absorberschichten einsetzen, wobei hinter die erste Absorptionsschicht eine zweite als Sicherheitsschicht geschaltet wird. Hierbei kann die Kapazität für das Alkanol in der ersten Absorberschicht voll ausgenutzt werden, da ein teilweises »Durchschlagen« des Alkanols durch die erste Schicht in der zweiten Schicht aufgefangen wird. Das »Durchschlagen« kann in bekannter Weise, beispielsweise durch gaschromatographische Messung oder Infrarot-Detektoren im austretenden Produktstrom, erkannt werden. Nach Beladung dieser ersten Absorptionsschicht wird diese Absorbereinheit zur Desorption in den Einsatz-Kohlenwasserstoffstrom umgeschaltet. An die Stelle dieser ersten Absorptionsschicht tritt nun die bisherige Sicherheits-Absorptionsschicht, und an die Stelle der bisherigen Sicherheits-Absorptionsschicht tritt eine frisch regenerierte Absorberschicht.

Der Gesamtprozeß des erfindungsgemäßen Verfahrens kann beispielsweise durch die folgende allgemeine Reaktionsgleichung beschrieben werden:

| | | | | |
|---|---|---|---|---|
| Gemisch A (tert.-Olefin, andere Olefine, Alkane, Aromaten) | + | Absorber mit Alkanol (zur Desorption) | + | Alkanol, stöchiometrisch zum tert.-Olefin |
| → Alkyl- tert.-alkyl- ether | + | Absorber mit alkanol- Überschuß | + | Gemisch B (andere Olefine, Alkane, Aromaten) |

Aus dieser Reaktionsgleichung ist ersichtlich, daß eine zum tert.-Olefin stöchiometrische Menge Alkanol zugespeist wird, daß aber infolge der Desorption des Alkanols vom Absorberharz im Reaktor überschüssiges Alkanol, bezogen auf die Menge des tert.-Olefins, vorhanden ist. Ebenso kann die zusätzliche Frischalkanolmenge überstöchiometrisch gewählt werden, um den Alkanolüberschuß noch weiter zu erhöhen. Dadurch wird ein quantitativer Umsatz des tert.-Olefins erzielt. Dieser Alkanol-Überschuß im Reaktor beträgt 1 bis 400 Gew.-% über die zur quantitativen Umsetzung des tert.-Olefins benötigte Menge Alkanol.

Für den Fall, daß, besonders bei tert.-Olefinen mit höherer C-Atomzahl, das Reaktionsgleichgewicht bei einem unvollständigen Umsatz des tert.-Olefins zum zugehörigen Ether liegt, beispielsweise bei nur 75—95% des vollständigen Umsatzes, kann es wirtschaftlich sinnvoll sein, weniger als 100 Mol-% Alkanol pro 100 Mol-% tert.-Olefin einzusetzen. Höhere Alkanolmengen beeinflussen im allgemeinen das genannte Gleichgewicht kaum, führen aber zu einem unvertretbar hohen Aufwand bei der Alkanol-Rückgewinnung. Dennoch wird bevorzugt eine Alkanolmenge eingesetzt, die oberhalb der zur Erreichung des genannten Gleichgewichts liegt. Liegt beispielsweise das Umwandlungsgleichgewicht eines tert.-Olefins in den zugehörigen Ether bei etwa 80 Mol-% des insgesamt vorhandenen tert.-Olefins, so kann es sinnvoll sein, etwa 90 Mol-% Alkanol, bezogen auf die Molzahl des tert.-Olefins, einzusetzen. Die übrig bleibenden etwa 10 Mol-% Alkanol können sodann erfindungsgemäß abgetrennt werden.

Als Alkanol für das erfindungsgemäße Verfahren sei ein aliphatischer, geradkettiger oder verzweigter Alkohol mit 1 bis 8, bevorzugt 1 bis 4, besonders bevorzugt 1 oder 2 Kohlenstoffatomen genannt. Beispiele hierfür sind Methanol, Ethanol, Propanol, Isopropanol, Butanol, Isobutanol, geradkettiges oder verzweigtes Pentanol, Hexanol, Heptanol oder Octanol.

Als Gemisch A der obigen Reaktionsgleichung und damit als Einsatzstrom für die erfindungsgemäße Veretherung kommen alle Kohlenwasserstoffströme in Frage, die mindestens 1 tert.-Olefin enthalten. Solche tert.-Olefine haben beispielsweise 4 bis 10, bevorzugt 4 bis 6, besonders bevorzugt 4 oder 5 Kohlenstoffatome, wie i-Buten, tert.-Penten, tert.-Hexen, tert.-Octen oder tert.-Decen. Einsatzströme, die mindestens eines der genannten tert.-Olefine enthalten und damit erfindungsgemäß einsetzbar sind, können beispielsweise ein Roh-C₄-Schnitt oder ein C₄-Raffinat I (nach der Butadien-Extraktion) aus einem thermischen Cracker oder ein FCC-Rohproduktstrom aus einem Fluid-Catalytic-Cracker

3

**0 068 218**

(FCC) oder ein $C_4/C_5$-LCC-Strom eines Catalytic-Crackers (LCC = Light Catalytic Cracker) sein. Selbstverständlich kann der FCC-Rohprodukt-Strom vorher in einen $C_4$-FCC-Strom und einen $C_5$-FCC-Strom aufgeteilt worden sein. Ebenso geeignet ist ein $C_5$-Strom eines thermischen Crackers, beispielsweise ein Destillationsvorlauf bei der Aromatengewinnung. Weitere geeignete Produktströme sind solche einer n-Buten-Skelettisomerisierung oder einer i-Butan-Dehydrierung, in denen sich i-Buten vorfindet. Für den Fall, daß Kohlenwasserstoffgemische, die mehr als 1 tert.-Olefin enthalten, eingesetzt werden, können die Gemische mehrerer Alkyl-tert.-alkylether entstehen. Diese können sowohl als Gemische als auch nach destillativer Auftrennung in Form ihrer Einzelkomponenten weitere Verwendung finden.

Solche Gemische A enthalten beispielsweise 0,5 bis 80 Gew.-% eines oder mehrerer tert.-Olefine.

Für die erfindungsgemäße Absorption und Desorption liegt das zu behandelnde Stoffgemisch stets in flüssiger Phase vor. Damit lassen sich die Absorption und Desorption des Alkanols mit der ebenfalls in flüssiger Phase durchgeführten Veretherung ohne energetisch aufwendige Phasenwechsel durchführen. Für den Fall, daß ein Teilstrom der Veretherungsprodukte, der vom Alkanol befreit werden soll, durch destillative Trennung als Kopfprodukt anfällt, wird dieser vor der Alkanol-Absorption kondensiert, eine Operation, die zur weiteren Handhabung ohnehin notwendig wäre. Zur Einstellung einer flüssigen Phase wird, besonders bei tiefer siedenden Gemischen oder Anteilen darin, bei tieferen Temperaturen und/oder höheren Drucken gearbeitet. Jedoch kann bei höhersiedenden Anteilen auch bei höheren Temperaturen oder niedrigeren Drucken gearbeitet werden.

Die Absorption des überschüssigen Alkanols kann beispielsweise im Bereich von 0 bis 60°C, bevorzugt 15−35°C durchgeführt werden. Als Druck kann beispielsweise 0,1 bis 20 bar, bevorzugt 1 bis 15 bar, gewählt werden. Die Desorption des Alkanols mit Hilfe des Einsatzgasstromes kann grundsätzlich unter den gleichen Bedingungen ablaufen wie die Absorption. Dies gilt vor allem dann, wenn der Einsatzstrom mit dem tert.-Olefin wesentlich größer ist als der Abgasstrom ohne dieses tert.-Olefin. Im allgemeinen ist es wünschenswert, die Desorptionszeit kleiner als die Absorptionszeit zu halten. In diesem Fall wird die Desorption beispielsweise bei einer Temperatur von 20 bis 100°C, bevorzugt 30 bis 60°C, durchgeführt. Als Druck für die Desorption sei beispielsweise 3 bis 30 bar, bevorzugt 5 bis 15 bar, genannt.

Durch geeignete Auswahl der Temperaturen und Drucke für die Absorption und Desorption innerhalb der genannten Bereiche wird die Desorptionsgeschwindigkeit auf den 0,5- bis 10fachen, vorzugsweise den 1,5- bis 4fachen Wert der Absorptionsgeschwindigkeit eingestellt. Eine höhere Desorptionsgeschwindigkeit kann beispielsweise durch Einstellen einer höheren Temperatur, verglichen mit der Absorption, erreicht werden.

Beispielsweise muß für den Fall, daß aus dem Veretherungsgemisch zunächst ein alkanolfreier Teilstrom (Ether oder Restgas) abgetrennt wird, nur der verbleibende alkanolhaltige Teilstrom zur Alkanol-Entfernung über das Absorberharz geleitet werden. In diesem Fall ist der Einsatzstrom, beispielsweise durch die Anwesenheit des tert.-Olefins größer als der beschriebene verbleibende Teilstrom und erzeugt bei der Desorption eine größere WHSV als der verbleibende Teilstrom bei der Absorption. Außerdem hat sich überraschenderweise gezeigt, daß die Desorptionsgeschwindigkeit für das Alkanol mit dem Gehalt an tertiären Olefinen im Spülstrom steigt, so daß zum Einstellen einer höheren Desorptionsgeschwindigkeit die Wahl einer höheren Temperatur gegebenenfalls entbehrlich ist. Insbesondere für eine kontinuierliche Durchführung des erfindungsgemäßen Verfahrens kann dadurch sichergestellt werden, daß die Desorptionszeit kleiner ist als die Absorptionszeit und damit bei Erschöpfung des Absorptionsfilters immer eine frische, regenerierte Absorberschicht zur Verfügung steht. Die Belastung der Absorberschicht zur Absorption und zur Desorption wird innerhalb des Bereiches einer stündlichen Raumgeschwindigkeit WHSV (Weight Hourly Space Velocity) von 0,1 bis 100, bevorzugt von 0,5 bis 20, besonders bevorzugt von 1 bis 15 kg Gasstrom pro kg Absorber pro Stunde gewählt. Vorteilhaft wählt man für die Desorption eine höhere WHSV als für die Absorption. Die Desorption kann bei der gleichen niedrigen Temperatur wie die Absorption erfolgen und dennoch zugunsten einer kontinuierlichen Fahrweise schneller sein als die Absorption. Diese Wahl tieferer Temperaturen sichert insbesondere eine energiesparende Betriebsweise.

Das aus der Reaktionszone ablaufende Produktgemisch oder ein in der Aufarbeitung anfallender Teilstrom können eine Alkanolkonzentration zwischen 0,01 und 90 Gew.-%, bevorzugt von 1 bis 60 Gew.-%, bezogen auf den Gesamtstrom, aufweisen.

Als Absorberharz für das erfindungsgemäße Verfahren seien beispielsweise synthetische Ionenaustauscher mit Kationen oder Anionen austauschenden Gruppen oder Gemische solcher Ionenaustauscher genannt.

Solche Ionenaustauscher haben beispielsweise eine Matrix auf der Basis von vernetzten Styrolpolymeren. Als Vernetzer seien beispielsweise Divinylbenzol, Trivinylbenzol oder Trivinylcyclohexan in einer Menge von etwa 0,3−80 Gew.-%, bevorzugt 1−65 Gew.-%, besonders bevorzugt 2−50 Gew.-%, bezogen auf die Gesamtmenge der Comonomeren, genannt. Die Matrix kann jedoch auch ein Phenol-Formaldehyd-Kondensat, ein (Meth)Acrylharz oder ein Epichlorhydrin/Polyamin-Kondensat, jeweils in vernetzter Form, sein. Solche vernetzten Matrices können in der gelförmigen oder der makroporösen Form eingesetzt werden.

Als austauschende Gruppen auf diesen Matrices seien beispielsweise genannt: Sulfonsäuregruppen, Phosphonsäuregruppen und Carboxylgruppen, jeweils in der $H^+$-Form oder in der Metallionen

4

Form. Hierzu seien Metallionen aus allen Gruppen des Periodensystems (Mendelejeff) genannt, beispielsweise $Li^+$, $Na^+$, $K^+$, $Rb^+$, $Cs^+$, $NH_4^+$, $Cu^+$, $Ag^+$, $Mg^{++}$, $Ca^{++}$, $Ba^{++}$, $Zn^{++}$, $Al^{+++}$, $Sn^{++}$, $Pb^{++}$, $Ce^{4+}$, $UO_2^{++}$, $Cr^{+++}$, $Co^{++}$, $Ni^{++}$, $Fe^{++}$, $Fe^{+++}$ oder $Pd^{++}$. Bevorzugt seien genannt: $Na^+$, $K^+$, $NH_4^+$, $Ca^{++}$, $Fe^{+++}$, besonders bevorzugt $Na^+$.

Weitere austauschende Gruppen können beispielsweise sein: $-NR_3^+$, wie $-N(CH_3)_3^+$ oder $-N(CH_3)_2CH_2CH_2OH^+$, oder $-NR_2$, wie $-N(CH_3)_2$ sowie N-Oxid-gruppen. Solche N-haltigen Gruppen können als austauschbares Gegenion beispielsweise das $OH^-$, $Cl^-$ oder $SO_4$-Ion enthalten. Solche Anionenaustauscher mit der Gruppe $-N(CH_3)_3^+$ sind dem Fachmann als solche des Typs I und mit der Gruppe $-N(CH_3)_2CH_2CH_2OH^+$ als solche des Typs II bekannt.

Ionenaustauscher der beschriebenen Art haben beispielsweise Totalkapazitäten für den Ionenaustausch von etwa 0,5–6 val/l Harz. Solche Harze und ihre Herstellungsverfahren sind seit langem bekannt (Ion Exchange, F. Helfferich, McGraw-Hill Book-Company, New York 1962; Ullmanns Enzyklopädie der technischen Chemie, 4. Aufl., Band 13, S. 279 ff., Verlag Chemie 1977).

Beispiele für erfindungsgemäß einsetzbare Harze sind: Sulfonsäuregruppen enthaltende gelförmige Styrol-Divinylbenzol-Harze, Sulfonsäuregruppen enthaltende makroporöse Styrol-Divinylbenzol-Harze, Carboxylgruppen enthaltende gelförmige oder makroporöse (Meth)Acrylsäure-Divinylbenzol-Harze, gelförmige oder makroporöse Styrol-Divinylbenzol-Anionenaustauscher vom Typ I oder II, makroporöse oder gelförmige Styrol-Divinylbenzol-Harze mit $-N(CH_3)_2$-Gruppen, schwach basische, makroporöse Harze des Acrylamid-Divinylbenzol-Typs, stark basische, makroporöse Harze des Acrylamid-Divinylbenzol-Typs oder Sulfonsäuregruppen enthaltende vernetzte Phenol-Formaldehyd-Harze. Die Aufzählung ist nicht vollständig und stellt keine Einschränkung auf die genannten Harze dar. Viele dieser Harze sind Handelsprodukte verschiedener Hersteller.

Das erfindungsgemäße Verfahren kann in mehreren Varianten durchgeführt werden, die beispielhaft mit Hilfe der anhängenden Zeichnungen erläutert werden können.

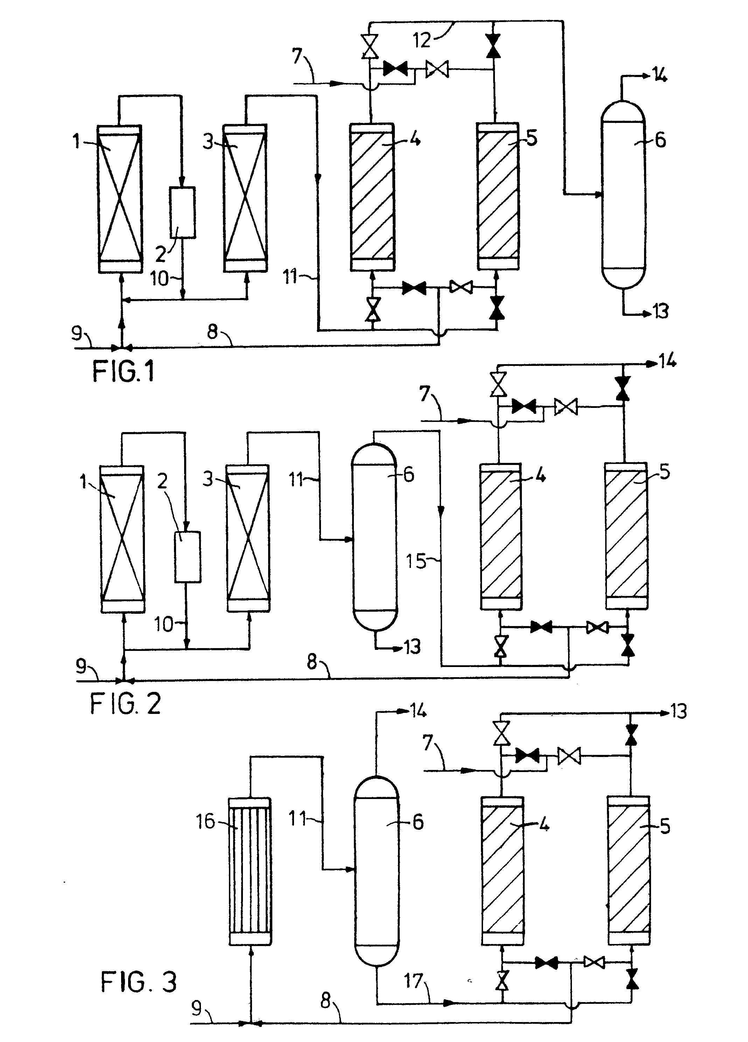

In Fig. 1 bedeuten die Positionen 1 und 3 zwei Veretherungsreaktoren, in denen der Veretherungskatalysator als Festbett angeordnet ist. Als Veretherungskatalysator wird im allgemeinen ein an sich bekannter Kationenaustauscher in der $H^+$-Form eingesetzt, beispielsweise ein Styrol-Divinylbenzol-Polymerisat, das Sulfonsäuregruppen trägt. Die Veretherungsreaktoren werden im Beispiel der Fig. 1 so betrieben, daß in 1 nur ein Teil der Veretherung durchgeführt wird, während in 3 diese Reaktion vervollständigt wird. Zur Abführung der positiven Wärmetönung dient ein Kühler 2. Das den Kühler 2 verlassende Reaktionsgemisch wird aufgeteilt und zum Teil nach 1, zum anderen Teil nach 3 zur Vervollständigung der Reaktion weitergeführt. Das umgesetzte Reaktionsgemisch, das nach der Veretherung weitgehend frei von tert.-Olefinen ist, wird nunmehr in eine Absorberschicht 4 zur Alkanol-Entfernung geleitet. Das die Absorberschicht 4 verlassende, vom Alkanol befreite Reaktionsgemisch 12 wird nunmehr in einen sogenannten Debutanizer 6 eingespeist, in dem es in den gewünschten reinen Alkyl-tert.-alkylether 13 als Sumpfprodukt und ein weitgehend vom Isoolefin und vom Alkanol befreites Kohlenwasserstoff-Raffinat 14 als Kopfprodukt aufgetrennt wird. Der das Isoolefin enthaltende Einsatzstrom 7 wird über eine Absorberschicht 5 geleitet, die von einem vorangegangenen Durchgang des Verfahrens das überschüssige Alkanol absorbiert enthält. Der Einsatzstrom belädt sich beim Durchgang durch die Absorberschicht 5 mit diesem Alkanol und wird als Strom 8 in den Reaktor 1 geleitet. Das in der Veretherung verbrauchte Alkanol wird diesem Strom 8 vor seinem Eintritt in den Reaktor als Strom 9 zur Ergänzung zugemischt.

Selbstverständlich läßt sich die in Fig. 1 als Beispiel gezeigte Variante noch dadurch ändern, daß der Veretherungskatalysator in nur einem Reaktor angeordnet ist. Dabei wird die Veretherung in diesem einen Reaktor vollständig durchgeführt und das diesen Reaktor verlassende Reaktionsgemisch wird, gegebenenfalls über einen Kühler, zum Teil in diesen einen Veretherungsreaktor als Verdünnung des Einsatzstromes zurückgeführt und nur ein Teil des vollständig veretherten Reaktionsgemisches zur Alkanolentfernung auf die Absorberschicht 4 gegeben. Selbstverständlich läßt sich anstelle eines Festbettreaktors auch ein Röhrenreaktor zur Veretherung einsetzen, bei dem ein um die Rohre fließendes Kühlmedium für die Abführung der Reaktionswärme sorgt.

Nach weitgehender Beladung der Absorberschicht 4 mit dem zu entfernenden Alkanol wird sodann durch eine Umschaltung der Ventile oberhalb und unterhalb der Absorberschichten 4 und 5 deren Funktion getauscht, so daß nunmehr 5 zur Alkanolentfernung aus dem fertigen Reaktionsgemisch dient und 4 zur Abgabe von absorbiertem Alkanol an das Einsatzgasgemisch dient. Zu diesem Zweck sind oberhalb und unterhalb von 4 und 5 Ventile angeordnet, von denen die in Fig. 1 schwarz ausgelegten geschlossen sind und die in Fig. 1 weiß ausgelegten geöffnet sind. Beim Umschalten der Funktion von 4 und 5 werden sodann die bisher geschlossenen Ventile geöffnet und die bisher geöffneten Ventile geschlossen. Dies kann beispielsweise in einer dem Fachmann bekannten Weise automatisch und gleichzeitig geschehen, so daß ein kontinuierlicher Betrieb keine oder nur eine minimale Unterbrechung erfährt. Diese automatische Betätigung der Ventile kann in einer ebenfalls dem Fachmann bekannten Weise, beispielsweise von der Überwachung der Alkanolkonzentration im von der Absorberschicht ablaufenden Strom 12 gesteuert werden. Diese zur Steuerung der Ventile dienende Alkanolkonzentration kann von der gewünschten Spezifikation, d. h. der in den Endprodukten zulässigen höchsten Alkanolkonzentration, abhängig gemacht werden. Wie bereits oben beschrieben, kann der zur Absorption dienenden Absorberschicht bei hohen Anforderungen an die Alkanolfreiheit der Endprodukte ein Sicherheits-Absorberfilter nachgeschaltet werden, das in der ebenfalls oben beschriebenen Weise in den Zyklus der Absorption und Desorption mit einbezogen werden kann.

Selbstverständlich kann auch die in Fig. 1 als einstufig dargestellte Destillation im Debutanizer 6 mehrstufig ausgeführt werden, falls die Lage der Siedepunkte des zu gewinnenden Alkyl-tert.-alkylethers und des zu gewinnenden Rest-Raffinats dies erforderlich machen.

Fig. 2 zeigt eine weitere mögliche Variante des erfindungsgemäßen Verfahrens. Die Bedeutung der Positionen 1 bis 14 ist in Fig. 2 die gleiche wie in Fig. 1. Während die in Fig. 1 aufgezeigte Verfahrensvariante die Reihenfolge Veretherungsreaktion-Alkanolentfernung-Destillation aufzeigte, wird in Fig. 2 der Verfahrensablauf Veretherungsreaktion-Destillation-Alkanolentfernung vorgestellt. Das aus dem Nachreaktor 3 ablaufende, weitgehend vom tert.-Olefin befreite Reaktionsgemisch tritt also zunächst in den Debutanizer 6 ein, in dem als Sumpfprodukt 13 der gewünschte Alkyl-tert.-alkylether anfällt und das über Kopf gehende, vom Ether befreite, jedoch noch alkanolhaltige Restgemisch 15 erhalten wird, das dann nach Kondensation zur flüssigen Phase anschließend in der Absorberschicht 4, wie bereits weiter oben beschrieben, in das alkanolfreie Rest-Raffinat 14 umgewandelt wird.

Selbstverständlich gelten für die in Fig. 2 dargestellte Verfahrensvariante auch alle oben bereits erwähnten Abwandlungen bezüglich des Ersatzes der Festbett-Veretherungsreaktoren 1 und 3 durch nur einen Festbettreaktor oder durch einen Röhrenreaktor, ferner den Ersatz der einstufigen Destillation im Debutanizer 6 durch eine mehrstufige und ferner die Ergänzung des auf Absorption eingerichteten Absorbers 4 durch ein dahintergeschaltetes Sicherheits-Absorptionsfilter. Insbesondere die Destillationsstufe im Debutanizer 6 kann noch die folgende Variation erfahren: Bei höheren Alkanolüberschüssen im Rahmen des oben angegebenen Bereiches kann die Destillation so eingerichtet werden, daß der ablaufende Alkyl-tert.-alkylether 13 etwas alkanolhaltig ist. Diese Variante wird insbesondere dann zur Anwendung gelangen können, wenn der Ether als Löse- oder Extrahiermittel vorgesehen ist und soweit die hierzu einzuhaltende Spezifikation bezüglich der Alkanolkonzentration dies erfordert. Die Destillation kann jedoch auch so geleitet werden, daß das gesamte Alkanol gemeinsam mit den Restkohlenwasserstoffen über Kopf abdestilliert wird. Bei dieser Variante des erfindungsgemäßen Verfahrens erweist es sich als vorteilhaft, die Alkanolmenge bei etwa 1,1 bis 1,2 Mol pro Mol umzusetzendes tert.-Olefin anzusetzen.

Die Umschaltung der in Fig. 2 dargestellten Absorberschichten 4 und 5 von Absorption auf Desorption und umgekehrt geschieht in gleicher Weise, wie im Zusammenhang mit Fig. 1 beschrieben.

Auch das Einsatzmaterial 7, das mit Alkanol beladene Einsatzmaterial 8, die Alkanolzuführung 9 und der Kreislaufstrom 10 zur Beherrschung der Reaktionswärme entsprechen vollständig der für Fig. 1 gegebenen Erläuterung.

In Fig. 3 wird wiederum die Reaktionsvariante mit der Reihenfolge Veretherungsreaktion-Destillation-Alkanolentfernung gezeigt, hier jedoch am Beispiel eines Röhrenreaktors 16, in dem die Veretherung bei höheren Alkanolüberschüssen betrieben wird. Der aus der Reaktion ablaufende und vom tert.-Olefin weitgehend befreite Reaktionsstrom wird im Debutanizer 6 so aufgetrennt, daß die Hauptmenge des überschüssigen Alkanols mit dem gewünschten Alkyl-tert.-alkylether im Sumpfprodukt 17 anfällt, das nunmehr in analoger Weise, wie bereits oben beschrieben, über die Absorberschicht 4 zur Alkanolentfernung und zur Gewinnung eines alkanolfreien Ethers 13 geschickt wird. Die Umschaltung der beiden Absorberschichten 4 und 5 und der zugehörigen Ventile oberhalb und unterhalb dieser Absorberschichten geschieht in der oben aufgezeigten Weise. Als Alkanolmenge sei für diese Variante beispielsweise eine Menge von 1,2 bis 6 Mol Alkanol pro Mol tert.-Olefin genannt. Außer im Sumpf 17 des Debutanizers 6 tritt auch im Kopfprodukt 14 Alkanol in der Größenordnung von ca. 0,5 bis 10 Gew.-% auf. Für den Fall, daß in diesem Kohlenwasserstoffraffinat der Alkanolgehalt stört, kann in ähnlicher Weise, wie in Fig. 2 gezeigt, das Kopfprodukt ebenfalls durch eine Absorberschicht geleitet werden, die selbstverständlich in die Desorption durch den Einsatzgasstrom einbezogen wird. Für diesen Fall werden also sowohl das Sumpfprodukt als auch das Kopfprodukt der Destillation über je eine Absorberschicht geleitet.

Es ist selbstverständlich, daß der stets bei der Destillation als Sumpfprodukt anfallende Ether bei höheren Anforderungen an die Reinheit einer weiteren Feindestillation unterworfen werden kann, wobei der Ether als Kopfprodukt entnommen wird.

Die Veretherungsreaktion erfolgt in einem der genannten Reaktortypen in bekannter Weise an einem sauren Kationenaustauscher, beispielsweise einem sulfonsäuregrupenhaltigen Styrol-Divinylbenzol-Polymerisat in fester oder schwebender Schicht bei einer Temperatur von 30 bis 120°C, bevorzugt 40 bis 90°C, und einem Druck von 1 bis 50 bar, bevorzugt 3 bis 20 bar mit einer stündlichen Kontaktbelastung (WHSV = Weight Hourly Space Velocity) von 0,1 bis 15 kg Gesamteinsatzstoffe pro kg Kationenaustauscher pro Stunde.

Als erfindungsgemäß herstellbare reine Alkyl-tert.-alkylether seien beispielsweise genannt:

Methyl-tert.-butylether (MTBE), Ethyl-tert.-butylether, Propyl-tert.-butylether,
Butyl-tert.-butylether, Hexyl-tert. butylether, Octyl-tert.-butylether,
Tert.-amyl-methylether (TAME), Tert.-amyl-ethylether, Tert.-amyl-butylether,
Tert.-amyl-octylether, Methyl-tert.-hexylether, Ethyl-tert.-hexylether,
Methyl tert. octylether, Ethyl-tert. octylether.

# 0 068 218

Solche Alkyl-tert.-alkylether enthalten restliches Alkanol in einer Menge unterhalb von 0,5 Gew.-%, beispielsweise <0,01 bis 0,5 Gew.-%, bevorzugt 0,01 bis 0,26 Gew.-%. Falls, wie oben beschrieben, ein festgelegter Restalkanol-Gehalt im Alkyl-tert.-alkylether erwünscht ist, kann dieser in der oben beschriebenen Weise eingestellt werden.

Als Kohlenstoffraffinate werden Gemische erhalten, die durch Anwendung des erfindungsgemäßen Verfahrens weitgehend frei von tert.-Olefinen sind, aber sonst den Einsatz-Kohlenwasserstoffgemischen entsprechen. Der Restgehalt an tert.-Olefinen liegt unter 8 Gew.-%, beispielsweise bei 0,1 bis 6 Gew.-%, bevorzugt bei 0,5 bis 4 Gew.-%. Der Gehalt an restlichem Alkanol liegt bei den für die Ether genannten Werten.

Das erfindungsgemäße Verfahren erlaubt es, für die Auftrennung des in der Veretherungsreaktion erhaltenen Reaktionsgemisches anstelle konventioneller vielstufiger Destillationsstufen in gegebenenfalls vielbödigen Kolonnen absorptive Aufarbeitungsstufen, kombiniert mit nur einer, im allgemeinen sehr einfach auszuführenden Destillationsstufe, anzuwenden. Dies bringt große Einsparungen bezüglich der Investitions- und Energiekosten mit sich.

Es ist überraschend, daß zum einen an den erfindungsgemäß einsetzbaren Absorberharzen die zur Veretherung im Überschuß eingesetzten Alkanole derart festgehalten werden, daß auch bei höheren Belastungen der Absorberschichten innerhalb der angegebenen Bereiche ein »Durchschlagen« des Alkanols verhindert werden kann, so daß spezifikationsgerechte Ether und Restgase erhalten werden können. Zum anderen ist es im Lichte der eingangs zitierten Literatur überraschend, daß das absorbierte Alkanol ohne drastische Behandlungsmethoden, wie Ausdämpfen oder Trocknen im Vakuum bei Temperaturen über 100°C entfernt werden kann, daß vielmehr diese Entfernung mit Hilfe des Reaktionseinsatzstromes erfolgt, so daß die Einbindung der Absorption und Desorption der Alkanole in ein kontinuierliches Verfahren durch bloßes Umschalten der Absorberschichten vorgenommen werden kann.

Da die Desorption des Alkanols durch die tertiären Olefine des Spülstroms gefördert wird, kann die Desorption gegebenenfalls sogar bei der Absorptionstemperatur erfolgen, so daß die regenerative Methanolentfernung nahezu ohne Energiebedarf bei einem gegebenen Temperaturniveau durchgeführt werden kann.

## Beispiel 1

Ein temperierbarer Durchlaufreaktor mit einer lichten Weite von 20 mm wurde mit einem makroporösen, sulfonsäuregruppenhaltigen Styrol-Divinylbenzol-Polymerisat (mit 18% Divinylbenzol vernetzt; Totalkapazität 1,4 val/l; Handelsprodukt Lewatit SPC 118 der Bayer AG) in der $H^+$-Form gefüllt, dessen Menge zur einzusetzenden Menge der Ausgangsstoffe so bemessen ist, daß eine Kontaktbelastung von WHSV = 1 (weight hourly space velocity) erreicht wird. Die Temperaturkontrolle erfolgt über Temperaturmeßstellen, die im Abstand von jeweils 100 mm am Reaktionsrohr angebracht sind. Bei einer Reaktionstemperatur von 40°C und einem über eine Druckhaltung geregelten Reaktionsdruck von 15 bar wurden 100 g $C_4$-Raffinat I/h (vgl. Tabelle I) und 26,95 g Methanol/h nach Durchlaufen einer Mischkammer über den Kontakt geleitet. Das den Reaktor verlassende Reaktionsprodukt wurde in einem gekühlten Abscheider zwischengelagert. Die gaschromatographisch bestimmte Zusammensetzung von Einsatzstrom und Produktstrom sind in der Tabelle I dargestellt.

Tabelle I

|  | Einsatzstrom [Gew.-%] | Produktstrom [Gew.-%] |
|---|---|---|
| i-Buten | 37,2 | 0,7 |
| n-Butene | 32,4 | 31,9 |
| Butane | 9,2 | 9,2 |
| Methanol | 21,2 | 0,9 |
| MTBE (Methyl-tert.-butylether) | — | 55,8 |
| Oligomere etc. | — | 1,5 |

Umsatz von i-Buten 98,1%.

## Beispiel 2

Die Methanolentfernung aus dem Produktstrom von Beispiel 1 erfolgt über 2 im Wechseltakt betriebene Stahlrohrreaktoren mit einer lichten Weite von 25 mm und einem Gesamtvolumen von 250 ml. Die Reaktoren haben äußere Mäntel, durch die über einen Thermostaten Wasser umläuft. Sie sind weiterhin mit Temperaturmeßstellen versehen. Der Druck wird über eine Druckhaltung auf 5 bar geregelt. Die Zudosierung des Produktstromes erfolgt von unten nach oben über eine Membrankolbenpumpe. Am Ausgang des Reaktors werden Momentan- und Durchschnittsproben gezogen und gaschromatografisch untersucht. In die im Wechseltakt betriebenen Reaktoren werden je 120 ml getrocknetes makroporöses, schwach basisches Styrol/Divinylbenzol-Harz mit Dimethylbenzylamin-Gruppen (Handelsprodukt Lewatit MP 62 der Bayer AG) eingefüllt (≙ 100 g wasserfeuchtes Harz).

Zur Methanolabsorption werden 1,2 l/h des Produktstromes aus Beispiel 1 (≙ 840 g/h) mit einem Methanolgehalt von 0,9 Gew.-% von unten nach oben über einen der beiden Absorber geleitet. Die Temperatur wird hierbei bei 20°C gehalten. Die Absorptionseffekte und ihre zeitliche Entwicklung über einen Zeitraum von 5 Stunden sind in der Tabelle II wiedergegeben.

Tabelle II

| t [h] | Beschickung [g] | Methanol im Eluat [Gew.-%] |
|---|---|---|
| 0,5 | 420 | 0,1 |
| 1 | 840 | 0,1 |
| 2 | 1680 | 0,1 |
| 3 | 2520 | 0,1 |
| 4 | 3360 | 0,2 |
| 4,5 | 3780 | 0,4 |
| 5 | 4200 | 0,7 |

Nach 5 Stunden wird der Produktstrom aus Beispiel 1 auf den 2. Absorber umgeschaltet, während der 1. Absorber mit 1,2 l/h $C_4$-Raffinat I (≙ 840 g/h) bei 50°C und 6 bar von unten nach oben gespült wird. Der Spülstrom hat folgende Zusammensetzung:

| | |
|---|---|
| i-Buten | 47,2 Gew.-%, |
| n-Butene | 41,1 Gew.-% und |
| Butane | 11,7 Gew.-%. |

Die Desorptionseffekte des Spülvorganges sind in der nachfolgenden Tabelle III dargestellt.

Tabelle III

| t [h] | Spülstrom [g] | Methanol im Eluat [Gew.-%] |
|---|---|---|
| 0,5 | 420 | 2,5 |
| 1,5 | 1260 | 1,4 |
| 2,5 | 2100 | 0,1 |

Der vom Desorptionsvorgang mit Methanol beladene Spülstrom wird anschließend nach Ergänzung um die zur Reaktion benötigte Methanolmenge in den Veretherungsreaktor eingeführt. Nach etwa 5 Stunden werden die beiden Absorptionsreaktoren wieder umgeschaltet und in diesem Wechseltakt kontinuierlich weiterbetrieben. Der vom Methanol weitgehend befreite Produktstrom nach der Absorption wird in eine Druckdestillationskolonne geleitet, in der bei ca. 5 bar die restlichen $C_4$-Kohlenwasserstoffe über Kopf als Raffinat II mit einem Methanolgehalt von 0,2 Gew.-% und im Sumpf ein 97gew.-%iger Methyl-tert.-butyl-ether (MTBE) mit etwa 2,5 Gew.-% Anteilen an $C_4$-Oligomeren gewonnen wird.

## Beispiel 3

Der Produktstrom aus Beispiel 1 wird in eine Druckdestillationskolonne geleitet, in der bei etwa 5 bar die restlichen $C_4$-Kohlenwasserstoffe über Kopf als Raffinat II mit einem Methanolgehalt von 2,1 Gew.-% und im Sumpf einen 97gew.-%igen MTBE mit 2,5 Gew.-% Oligomerenanteilen gewonnen wird.

Der Kopfstrom der Destillationsstufe wird zur kontinuierlichen absorptiven Methanolentfernung in die nachfolgend beschriebene Apparatur geleitet:

Die Absorptionsanlage besteht aus zwei parallel geschalteten, wechselweise zu betreibenden, temperierbaren Doppelmantelreaktoren von 140 cm Länge und einer lichten Weite von 25 mm, die mit jeweils 680 cm³ eines methanolfeuchten makroporösen, schwach basischen Styrol/Divinylbenzol-Harzes mit Dimethylbenzylamin-Gruppen (Handelsprodukt MP 62 der Bayer AG) in Kugelform (Durchmesser von 0,3 bis 1,5 mm) beschickt wird. Beide Reaktoren sind durch ein Rohrleitungssystem so miteinander verbunden, daß einerseits eine Absorption von Methanol bei 5 bar und 22° C aus einem $C_4$-Kohlenwasserstoffstrom (Raffinat II) und andererseits eine Desorption des auf dem Absorberharz befindlichen Methanols mit einem methanolfreien $C_4$-Kohlenwasserstoffstrom (Raffinat I) bei 50° C und 20 bar ermöglicht wird und anschließend die Umschaltung auf den jeweils umgekehrten Vorgang erfolgen kann. Die Methanolgehalte des Absorptions- und des Desorptionsstromes werden über einen kontinuierlich betriebenen Gaschromatographen überwacht. Da das Absorberharz in der methanolhaltigen Form eingesetzt wird, wird zunächst der Spülstrom, bestehend aus dem $C_4$-Raffinat I (i-Buten 47,2 Gew.-%, n-Butene 41,1 Gew.-% und Butane 11,7 Gew.-%) zur Desorption des Methanols über das Absorberharz geschickt. Die Desorption wird bei 50° C und 20 bar mit 1250 ml Spülstrom/h betrieben. Das den Desorptionsreaktor verlassende $C_4$-Raffinat I hat anfangs einen Methanolgehalt von mehr als 5 Gew.-%, der im Verlauf der ersten Stunde auf 0,1 Gew.-% absinkt. Nach 1½ Stunden einschließlich einer Aufheizzeit von ½ Stunde ist die Desorption der ersten Absorberharzschicht abgeschlossen. Nach dem Umschalten des Spülstroms auf die zweite Absorberharzschicht wird ein Produktstrom, der analog Beispiel 1 erhalten worden war, zur Methanolentfernung über die erste Absorberharzschicht geleitet. Dieser Produktstrom hat die folgende Zusammensetzung:

| | |
|---|---|
| i-Buten | 1,6 Gew.-% |
| n-Butene | 74,7 Gew.-% |
| Butane | 21,5 Gew.-% |
| Methanol | 2,1 Gew.-% |
| Rest | 0,1 Gew.-% |

Von diesem Produktstrom werden stündlich 750 ml bei 22° C und 5 bar über die Absorberharzschicht geleitet. Das den Absorptionsreaktor verlassende $C_4$-Raffinat II enthält während einer Zeit von 2½ Stunden weniger als 0,1 Gew.-% Methanol und erreicht erst nach 3 Stunden einen Methanolgehalt von 1 Gew.-%. Danach werden Spül- und Raffinat II-Strom an den beiden Absorptionsreaktoren umgeschaltet. Diese Umschaltungen werden mit einem Zeittakt von 3 Stunden über einen Zeitraum von 20 Tagen fortgeführt, ohne daß die Absortionseffekte sich ändern. Dabei wird folgende $C_4$-Raffinat II-Spezifikation erreicht:

| | |
|---|---|
| i-Buten | 1,7 Gew.-% |
| n-Butene | 76,2 Gew.-% |
| Butane | 21,9 Gew.-% |
| Methanol | 0,1 Gew.-% |
| Rest | 0,1 Gew.-% |

## Beispiel 4

Zwei hintereinandergeschaltete Rohrreaktoren mit einer lichten Weite von 20 mm werden mit je 140 g eines makroporösen sulfonsäuregruppenhaltigen sauren Styrol-Divinylbenzol-Kationentauscherharzes wie in Beispiel 1 gefüllt. Zwischen beiden Reaktoren ist eine Produktrückführungsabzweigung eingerichtet, so daß durch Einstellen eines Durchlaßventils und mit Hilfe einer Pumpe ein Teil des Produktstromes nach Durchlaufen eines Kühlers an den Reaktoreingang des ersten Reaktorrohres zurückgeführt werden kann. Die Temperaturkontrolle erfolgt über Temperaturmeßstellen, die im Ab-

**0 068 218**

stand von jeweils 100 mm am Reaktionsrohr angebracht sind. Bei einem eingestellten Rückführverhältnis von 2 : 1 (Rückführung zu Frischeinsatz) werden 100 g/h $C_4$-Raffinat I und 40 g/h Methanol bei 40 bis 70°C im ersten Reaktor und bei 40°C im zweiten Reaktor zur Reaktion gebracht. Die Zusammensetzungen von Einsatzstrom und Produktstrom sind in der nachfolgenden Tabelle IV dargestellt.

Tabelle IV

|  | Einsatzstrom [Gew.-%] | Produktstrom [Gew.-%] |
|---|---|---|
| i-Buten | 33,7 | 0,3 |
| n-Butene | 29,4 | 29,0 |
| Butane | 8,4 | 8,4 |
| Methanol | 28,5 | 9,8 |
| MTBE | — | 51,4 |
| Oligomere etc. | — | 1,1 |

Umsatz: 99,1% des i-Butens.

## Beispiel 5

Der Produktstrom aus Beispiel 4 wird in eine Druckdestillationskolonne geleitet, in der bei etwa 5 bar die restlichen $C_4$-Kohlenwasserstoffe mit etwa 2,5 Gew.-% Methanol über Kopf als Raffinat II und im Sumpf ein 83,8gew.-%iges MTBE mit 14,4 Gew.-% Methanol und etwa 1,8 Gew.-% Oligomerenanteilen gewonnen wird. Dieser Sumpfstrom wird bei 25°C mit einer WHSV (weight hourly space velocity) von 5, bezogen auf trockenes Absorberharz, über 30 g einer Absorberharzschicht aus dem in Beispiel 2 und 3 eingesetzten Ionenaustauscher geleitet. Die Versuchsanordnung entspricht der in Beispiel 2. Am Ausgang der Absorberharzschicht wird ein Stoffstrom erhalten, der nach gaschromatografischer Analyse wie folgt zusammengesetzt ist:

| MTBE | 96,8 Gew.-% |
|---|---|
| Methanol | 0,9 Gew.-% |
| nicht näher bestimmter Rest | 2,3 Gew.-% |

Nach $1^1/_2$ Stunden wird der Beschickungsstrom (das Sumpfprodukt) auf die zweite Absorberharzschicht umgeschaltet. Die Desorption des Methanols von der ersten Absorberharzschicht erfolgt nach den in Beispiel 2 angegebenen Bedingungen. Der Absorptions-Desorptions-Zyklus wurde mehrfach wiederholt.

## Beispiel 6

### a) Herstellung eines mit Palladium dotierten Kationenaustauschers

Entsprechend der DE-PS 1 113 570, Beispiel 3, wurde ein makroporöser Kationenaustauscher hergestellt. Der wasserfeuchten $H^+$-Form dieses Kationenaustauschers wurde im Batch so viel Palladiumacetat angeboten, daß 0,75 g Palladium pro Liter trockenes Harz nach Reduktion mit Wasserstoff auf den Kationenaustauscher vorhanden waren. Die bei der Behandlung mit Palladiumacetat freigesetzte Säure wurde mit 1gew.-%iger Natronlauge neutralisiert. Anschließend wurde der neutral gewaschene Kationenaustauscher 24 Stunden bei 100°C im Vakuum einer Wasserstrahlpumpe getrocknet. Das auf dem Kationenaustauscher befindliche Palladium wurde dann zwischen 90 und 100°C mit Wasserstoff bei einem Druck von 20 bis 25 bar innerhalb von 48 Stunden zum Metall reduziert.

### b) Veretherung

In der in Beispiel 1 beschriebenen Apparatur werden 100 g/h eines teilhydrierten $C_5$-Kohlenwasser-

stoffstromes eines thermischen Crackers (Aromatenvorlauf) mit 20 Gew.-% an Isoamylenen und 8,6 g/h Methanol an 110 g des unter a) beschriebenen Kationenaustauschers (0,75 g Pd pro Liter Kationenaustauscher) bei 70° C und 10 bar zur Reaktion gebracht. Die gaschromatografische Untersuchung des Produktstromes weist folgende Zusammensetzung aus:

| i-Amylene | 4,1 Gew.-% |
|---|---|
| TAME (Tert.-amyl-methylether) | 20,9 Gew.-% |
| Methanol | 1,2 Gew.-% |
| Rest C$_5$-KW | 72,1 Gew.-% |
| Höhere Ether + KW | 1,7 Gew.-% |

Umsatz der i-Amylene: 78 Gew.-%.

## Beispiel 7

Der Produktstrom aus Beispiel 6 wird in eine Destillationskolonne gegeben, in der TAME mit einer Reinheit von 95 Gew.-% mit 5 Gew.-% höheren Ethern und Kohlenwasserstoffen im Seitenstrom der Kolonne und die restlichen C$_5$-Kohlenwasserstoffe mit 1,6 Gew.-% Methanol als Kopfprodukt gewonnen werden. Dieser Kopfstrom wird bei 25° C mit einer WHSV von 20, bezogen auf trockenes Absorberharz, über 15 g des in Beispiel 2, 3 und 5 eingesetzten Ionenaustauschers geleitet. Die Apparatur ist die gleiche wie in Beispiel 2. Die Zusammensetzung des Destillations-Kopfstromes am Ausgang des Absorberharzes wird nach definierten Zeiten gaschromatografisch analysiert. Die Absorptionseffekte und ihre Entwicklung über einen Zeitraum von 3$^1$/$_2$ Stunden sind in der Tabelle V wiedergegeben.

Tabelle V

Beschickung:

| C$_5$-Kohlenwasserstoffe | 97,9 Gew.-% |
|---|---|
| Methanol | 1,6 Gew.-% |
| TAME | 0,5 Gew.-% |

Absorptionseffekt über 3,5 Stunden:

| t [h] | Methanol im Eluat [Gew.-%] |
|---|---|
| 0,5 | 0,1 |
| 1,0 | 0,1 |
| 1,5 | 0,1 |
| 2,0 | 0,1 |
| 2,5 | 0,1 |
| 3,0 | 0,1 |
| 3,5 | 0,2 |

Anschließend wird der Beschickungsstrom auf die zweite Absorberschicht umgeschaltet. Die Methanoldesorption von der ersten Absorberschicht erfolgt nach den in Beispiel 2 angegebenen Bedingungen. Der Absorptions-Desorptions-Zyklus wird fünfmal wiederholt, ohne daß die Methanolaufnahmekapazität nachläßt.

## Beispiel 8

In der in Beispiel 1 beschriebenen Apparatur werden 100 g/h eines teilhydrierten C$_5$-Kohlenwasserstoffstromes eines thermischen Crackers (Aromatenvorlauf) mit 50 Gew.-% an Isoamylen und 12,4 g/h Ethanol an einem mit Palladium dotierten stark sauren makroporösen Kationenaustauscher (0,75 g Pd pro Liter Harz) bei 70°C und 10 bar zur Reaktion gebracht. Die gaschromatografische Untersuchung des Produktstromes weist folgende Zusammensetzung aus:

| | |
|---|---|
| i-Amylene | 4,4 Gew.-% |
| TAEE (Tert.-amyl-ethylether) | 22,2 Gew.-% |
| Ethanol | 2,6 Gew.-% |
| Rest C$_5$-KW | 69,0 Gew.-% |
| Höhere Ether + KW | 1,8 Gew.-% |

Umsatz der i-Amylene: 75%.

Dieser Produktstrom wird bei 25°C mit einer WHSV von 20, bezogen auf trockenes Absorberharz, über 15 g des in Beispiel 2, 3, 5 und 7 eingesetzten Ionenaustauschers geleitet. Als Apparatur wird die in Beispiel 2 beschriebene verwendet.

Die Zusammensetzung des Absorberausgangsstromes wird nach definierten Zeiten gaschromatografisch analysiert. Die Absorptionseffekte und ihre Entwicklung über einen Zeitraum von 3$^1$/$_2$ h sind in der Tabelle VI wiedergegeben.

Tabelle VI

| t [h] | Ethanol im Eluat [Gew.-%] |
|---|---|
| 0,5 | 0,1 |
| 1,0 | 0,1 |
| 1,5 | 0,1 |
| 2,0 | 0,1 |
| 2,5 | 0,1 |
| 3,0 | 0,2 |
| 3,5 | 0,5 |

Anschließend wird der Beschickungsstrom auf die 2. Absorberharzschicht umgeschaltet. Die Desorption des Ethanols von der 1. Absorberharzschicht erfolgt nach den in Beispiel 2 angegebenen Bedingungen. Der Absorptions-Desorptions-Zyklus wird fünfmal wiederholt, ohne daß die Ethanolaufnahmekapazität nachläßt.

Beispiele 9–17

In der im Beispiel 2 beschriebenen Absorptionsapparatur wurde der C$_5$-Kohlenwasserstoffstrom, der als Kopfprodukt der in Beispiel 7 aufgeführten Destillation gewonnen wurde, über die unten angegebenen Absorberharze zur Methanolentfernung geleitet. Zur Desorption wurde der C$_5$-Einsatzstrom aus Beispiel 6 verwendet.

Die über Gaschromatographie-Messungen ermittelten Absorptions- und Desorptionseffekte an den verschiedenen Absorberharzen sind in der Tabelle VII dargestellt.

Die in den Beispielen eingesetzten Ionenaustauscher sind in

Beispiel 10:
SC 102, gelförmiges vernetztes Styrol/DVB-Harz (Divinylbenzol) mit Sulfonsäure-Gruppen, stark sauer, in der Na$^+$-Form.
Beispiel 11:
CNP 80, makroporöser, schwach saurer Kationenaustauscher vom Acrylsäure-Typ mit spezieller Vernetzung.
Beispiel 12:
DN H$^+$, Kondensationsharz auf Basis Phenol/Formaldehyd mit Sulfonsäuregruppen.

12

Beispiel 13:
SPC 118, wie SC 102 aber makroporös.

Beispiel 14:
MP 62, makroporöses, schwachbasisches Styrol/DVB Harz mit Dimethylbenzylamin-Gruppen.

Beispiel 15:
MP 500, stark basisches makroporöses Styrol/DVB-Harz vom Typ I.

Beispiel 16:
MP 504, stark basisches gelförmiges Styrol/DVB-Harz vom Typ I.

Beispiel 17:
M 600, stark basisches gelförmiges Styrol/DVB-Harz vom Typ II.

Beispiel 18:
XAD 12 Styrol/DVB-Ionenaustauscher mit N-Oxid-gruppen.

Die Harze der Beispiele 10 – 17 sind Handelsprodukte der Bayer AG, das Harz des Beispiels 18 ist Handelsprodukt der Rohm and Haas Comp.

Tabelle VII

Beschickungsstrom:
$C_5$-KW-Strom + 2% Methanol; T = 25°, p = 1 bar; LHSV = 5 (Liquid Hourly Space Velocity)

Spülstrom:
$C_5$-KW-Strom; T = 35°, p = 1 bar; LHSV = 5

Mengen:
A = 630 g; B = 1260 g; C = 1890 g

| MeOH-Kon- zentration in Eluat | Beispiel | | | | | | | | |
|---|---|---|---|---|---|---|---|---|---|
| | 9 | 10 | 11 | 12 | 13 | 14 | 15 | 16 | 17 |
| | Adsorbens | | | | | | | | |
| | SC 102 Gew.-% | CNP 80 Gew.-% | DNH+ Gew.-% | SPC 118 Gew.-% | MP 62 Gew.-% | MP 500 Gew.-% | MP 504 Gew.-% | M 600 Gew.-% | XAD 12 Gew.-% |
| **Absorption** | | | | | | | | | |
| B | 0,1 | 0,4 | 0,5 | 0,7 | 0,1 | 0,3 | 0,4 | 0,8 | 0,1 |
| **Desorption** | | | | | | | | | |
| A | 4,2 | 2,8 | 1,9 | 2,1 | 3,4 | 2,7 | 2,4 | 1,7 | 2,8 |
| B | 2,7 | 1,6 | 1,2 | 0,9 | 1,9 | 0,9 | 0,9 | 0,8 | 1,3 |
| C | 0,5 | 0,3 | 0,2 | 0,1 | 0,5 | 0,3 | 0,2 | 0,3 | 0,4 |
| **Absorption** | | | | | | | | | |
| A | 0,1 | 0,4 | 0,3 | 0,4 | 0,1 | 0,1 | 0,2 | 0,4 | 0,1 |
| B | 0,1 | 0,6 | 0,5 | 0,6 | 0,1 | 0,4 | 0,4 | 0,7 | 0,1 |
| C | 0,4 | 0,8 | 0,7 | 1,0 | 0,1 | 1,0 | 0,9 | 1,2 | 0,1 |
| **Absorption** | | | | | | | | | |
| A | 4,1 | 3,7 | 2,0 | 1,8 | 3,8 | 3,0 | 2,5 | 1,4 | 2,8 |
| B | 2,4 | 1,5 | 1,1 | 0,6 | 2,6 | 1,7 | 0,8 | 0,5 | 1,0 |
| C | 0,7 | 0,4 | 0,3 | 0,3 | 0,5 | 0,5 | 0,3 | 0,2 | 0,5 |
| **Absorption** | | | | | | | | | |
| A | 0,1 | 0,5 | 0,4 | 0,6 | 0,1 | 0,1 | 0,2 | 0,4 | 0,1 |
| B | 0,4 | 0,8 | 0,9 | 0,8 | 0,1 | 0,5 | 0,7 | 1,0 | 0,1 |
| C | 0,6 | 1,0 | 1,1 | 1,2 | 0,2 | 0,8 | 1,0 | 1,2 | 0,3 |

**0 068 218**

## Patentansprüche

1. Verfahren zur Herstellung von reinen Alkyl-tert.-alkylethern und weitgehend vom tert.-Olefin und vom Alkanol befreiten Kohlenwasserstoff-Raffinaten durch Umsetzung eines Alkanols und mindestens ein tert.-Olefin enthaltenden Kohlenstoffgemischen an sauren Kationenaustauschern, dadurch gekennzeichnet, daß man überschüssiges, nicht-umgesetztes Alkanol aus dem aus der Reaktionszone ablaufenden Produktgemisch oder aus seinen in der Aufarbeitung anfallenden Teilströmen durch Absorption an einem synthetischen Ionenaustauscher mit Kationen oder Anionen austauschenden Gruppen oder Gemische solcher Ionenaustauscher als Absorberharz entfernt und das in die Reaktionszone einzuführende Alkanol mindestens teilweise dem hiermit beladenen Absorberharz durch Desorption mit Hilfe des mindestens 1 tert.-Olefin enthaltenden Kohlenwasserstoffgemisches entnimmt, wobei die in der Absorption und Desorption behandelten Stoffgemische in flüssiger Phase vorliegen.

2. Verfahren nach Anspruch 1, dadurch gekennzeichnet, daß die Absorption bei 0 bis 60°C und einem Druck von 0,1 bis 20 bar vorgenommen wird.

3. Verfahren nach Anspruch 1, dadurch gekennzeichnet, daß die Desorption bei 20 bis 100°C und einem Druck von 3 bis 30 bar, bevorzugt 5 bis 15 bar, durchgeführt wird.

4. Verfahren nach Ansprüchen 1 bis 3, dadurch gekennzeichnet, daß das zur Absorption und Desorption eingesetzte Absorberharz auf mindestens 2 Schichten aufgeteilt wird, von denen mindestens eine weitgehend alkanolfreie Schicht zur Absorption dient, nach weitgehender Beladung durch eine weitere alkanolfreie Schicht zur weiteren Absorption ersetzt wird, während die erste beladene Schicht zur Desorption in den Einsatz-Kohlenwasserstoffstrom zur Reaktion geschaltet wird und nach weitgehender Desorption des Alkanols wieder als Absorptionsschicht eingesetzt wird.

5. Verfahren nach Ansprüchen 1 bis 4, dadurch gekennzeichnet, daß die Desorptionsgeschwindigkeit auf den 0,5- bis 10fachen Wert der Absorptionsgeschwindigkeit eingestellt wird.

6. Verfahren nach Ansprüchen 1 bis 5, dadurch gekennzeichnet, daß die Absorptionsschicht und die Desorptionsschicht mit einer stündlichen Raumgeschwindigkeit WHSV (Weight Hourly Space Velocity) von 0,1 bis 100 kg Reaktionsgemisch pro kg Absorber pro Stunde belastet werden.

7. Verfahren nach Ansprüchen 1 bis 6, dadurch gekennzeichnet, daß zur Desorption eine höhere WSHV als zur Absorption eingestellt wird.

8. Verfahren nach Ansprüchen 1 bis 7, dadurch gekennzeichnet, daß bei der Absorption eine Alkanolkonzentration von 0,01 bis 90 Gew.-%, bezogen auf den gesamten Beschickungsstrom, vorliegt.

9. Verfahren nach Ansprüchen 1 bis 8, dadurch gekennzeichnet, daß das den Veretherungsreaktor verlassende Reaktionsgemisch zuerst an einem Absorberharz vom überschüssigen Alkanol befreit wird und danach durch Destillation in den Alkyl-tert.-alkylether und das Restgas getrennt wird.

10. Verfahren nach Ansprüchen 1 bis 8, dadurch gekennzeichnet, daß das den Veretherungsreaktor verlassende Reaktionsgemisch zuerst durch Destillation in den Alkyl-tert.-alkylester und in das Restgas getrennt wird, wobei mindestens einer der durch die Destillation erhaltenen Teilströme alkanolhaltig ist, und danach den oder die alkanolhaltigen Teilströme an einem Absorberharz vom Alkanol trennt.

## Claims

1. Process for the preparation of pure alkyl tert.-alkyl ethers and hydrocarbon raffinates which are largely free from tert.-olefine and from alkanol by reaction, on acid cation exchangers, of an alkanol and carbon mixtures containing at least one tert.-olefine, characterised in that excess, unreacted alkanol is removed from the product mixture leaving the reaction zone or from its part streams produced in the working-up, by absorption on a synthetic ion exchanger containing groups which exchange cations or anions, or mixtures of such ion exchangers as the absorber resin, and the alkanol to be introduced into the reaction zone is removed at least partly from the absorber resin charged with this alkanol, by means of desorption with the aid of the hydrocarbon mixture containing at least 1 tert.-olefine, the substance mixtures treated in the absorption and desorption being in the liquid phase.

2. Process according to Claim 1, characterised in that the absorption is carried out at 0 to 60°C and under a pressure of 0.1 to 20 bar.

3. Process according to Claim 1, characterised in that the desorption is carried out at 20 to 100°C and under a pressure of 3 to 30 bar, preferably 5 to 15 bar.

4. Process according to Claims 1 to 3, characterised in that the absorber resin used for the absorption and desorption is divided into at least 2 layers, of which at least one largely alkanol-free layer is used for the absorption, and, after being extensively charged, is replaced by a further alkanol-free layer for further absorption, while the first charged layer is switched, for desorption, to the starting hydrocarbon stream for the reaction, and after extensive desorption of the alkanol is used again as the absorption layer.

5. Process according to Claims 1 to 4, characterised in that the rate of desorption is adjusted to be 0.5 to 10 times the value of the rate of absorption.

6. Process according to Claims 1 to 5, characterised in that the absorption layer and the desorption layer are charged with an hourly space velocity WHSV (weight hourly space velocity) of 0.1 to 100 kg of reaction mixture per kg of absorber per hour.

7. Process according to Claims 1 to 6, characterised in that a higher WHSV is adjusted for the desorption than for the absorption.

8. Process according to Claims 1 to 7, characterised in that an alkanol concentration of 0.01 to 90% by weight, based on the total charging stream, is present during absorption.

9. Process according to Claims 1 to 8, characterised in that the reaction mixture leaving the etherification reactor is first freed, on an absorber resin, from the excess alkanol, and is then separated into the alkyl tert.-alkyl ether and the residual gas by distillation.

10. Process according to Claims 1 to 8, characterised in that the reaction mixture leaving the etherification reactor is first separated into the alkyl tert.-alkyl ether and the residual gas by distillation, at least one of the part streams obtained by the distillation containing alkanol, and then the part stream or streams containing alkanol is/are separated from the alkanol, on an absorber resin.

## Revendications

1. Procédé de préparation d'éthers alkyl-tert.-alkyliques purs et de raffinats d'hydrocarbures essentiellement libérés de l'oléfine tertiaire et de l'alcanol, par réaction d'un alcanol et de mélanges d'hydrocarbures contenant au moins une oléfine tertiaire, sur des échangeurs de cations acides, caractérisé en ce qu'on élimine l'alcanol en excès n'ayant pas réagi du mélange de produits s'écoulant de la zone réactionnelle ou de ses courants partiels se formant lors du traitement, par absorption sur un échangeur d'ions synthétique comportant des groupes échangeant des cations ou des anions ou des mélanges de tels échangeurs d'ions comme résine absorbante et on retire au moins partiellement l'alcanol devant être introduit dans la zone réactionnelle de la résine absorbante qui en est chargée, par désorption à l'aide du mélange d'hydrocarbures contenant au moins une oléfine tertiaire, les mélanges de matières traités lors de l'absorption et de la désorption étant présents en phase liquide.

2. Procédé suivant la revendication 1, caractérisé en ce qu'on effectue l'absorption à une température de 0 à 60° C et sous une pression de 0,1 à 20 bars.

3. Procédé suivant la revendication 1, caractérisé en ce qu'on effectue la désorption à une température de 20 à 100° C et sous une pression de 3 à 30 bars, de préférence, de 5 à 15 bars.

4. Procédé suivant les revendications 1 à 3, caractérisé en ce qu'on répartit la résine absorbante utilisée pour l'absorption et la désorption au moins sur deux couches dont au moins une essentiellement exempte d'alcanol sert à l'absorption et est, lorsqu'elle est essentiellement chargée, remplacée par une autre couche exempte d'alcanol en vue de l'absorption complémentaire, tandis que la première couche chargée est mise en service pour la réaction en vue de la désorption dans le courant d'hydrocarbures de charge et est réutilisée comme couche d'absorption lorsque l'alcanol est essentiellement désorbé.

5. Procédé suivant les revendications 1 à 4, caractérisé en ce que la vitesse de désorption est réglée à 0,5 – 10 fois la valeur de la vitesse d'absorption.

6. Procédé suivant les revendications 1 à 5, caractérisé en ce que la couche d'absorption et la couche de désorption sont sollicitées par une vitesse spatiale horaire en poids de 0,1 à 100 kg de mélange réactionnel/kg d'absorbant/heure.

7. Procédé suivant les revendications 1 à 6, caractérisé en ce que, pour la désorption, on règle une vitesse spatiale horaire en poids supérieure à celle prévue pour l'absorption.

8. Procédé suivant les revendications 1 à 7, caractérisé en ce que, lors de l'absorption, il y a une concentration en alcanol de 0,01 à 90% en poids, calculé sur tout le courant de charge.

9. Procédé suivant les revendications 1 à 8, caractérisé en ce que le mélange réactionnel quittant le réacteur d'éthérification est tout d'abord libéré de l'alcanol en excès sur une résine absorbante, pour être ensuite séparé, par distillation, en éthers alkyl-tert.-alkyliques et en gaz résiduaire.

10. Procédé suivant les revendications 1 à 8, caractérisé en ce que le mélange réactionnel quittant le réacteur d'éthérification est tout d'abord séparé, par distillation, en éthers alkyl-tert.-alkyliques et en gaz résiduaire, au moins un des courants partiels obtenus par la distillation contenant l'alcanol, tandis que le ou les courants partiels contenant l'alcanol sont ensuite séparés de ce dernier sur une résine absorbante.

FIG.1

FIG. 2

FIG. 3